# EUROPEAN PATENT SPECIFICATION

(11) **EP 0 970 377 B1**
(45) Date of publication and mention of the grant of the patent: **11.02.2004**
(21) Application number: 98912619.8
(22) Date of filing: 23.03.1998
(51) Int. Cl.: G01N 33/574, C07K 16/18

(54) **DIAGNOSIS OF EPITHELIAL CELL ABNORMALITIES**
DIAGNOSE VON EPITHELZELLABNORMITÄTEN
DEPISTAGE D'ANOMALIES DANS DES CELLULES EPITHELIALES

(30) Priority: 21.03.1997 GB 9705949
(43) Date of publication of application: 12.01.2000
(73) Proprietor: Europroteome AG, 16761 Hennigsdorf (DE)
(72) Inventor: HOCHSTRASSER, Denis, François, CH-1245 Collonge-Bellerive (CH); REYMOND, Marc, André, CH-1222 Vesenaz (CH); PEINADO, Miguel Angel Inst. de Recerca Oncologica, E-08907 L'Hospitalet (ES)
(74) Representative: Ziebig, Marlene, Dr. Dipl.-Chem.
(86) International application number: PCT/GB1998/000881
(87) International publication number: WO 1998/043091

(56) References cited:
- WO-A-97/09600
- WO-A-97/37226
- US-A- 5 547 928
- REYMOND ET AL: "Standardized characterization of gene expression in human colorectal epithelium by two-dimensional electrophoresis" ELECTROPHORESIS, vol. 18, no. 5, December 1997, pages 2842-2848, XP002071492
- REYMOND M A ET AL: "Specific sample preparation in colorectal cancer." ELECTROPHORESIS, vol. 18, no. 3-4, - 1997 pages 622-624, XP002072104

## Description

### Background of the invention

### 1. Field of the invention

This invention relates to the diagnosis of tumours and other abnormalities of body cells.

### 2. Description of the related art

There is a need for improved methods of diagnosis of tumours, particularly to determine whether there is any residual disease after surgery or therapy has taken place. This is particularly necessary to detect metastases. The present invention relates specifically to epithelial tumours and other abnormalities of epithelial cells. The term "diagnosis" as used herein includes the obtaining of any kind of information relating to the existence of or condition of a tumour or other abnormality, and includes prognosis.

In clinical practice, the TNM (tumour node metastasis) staging system, "TNM classification of malignant tumours", UICC, Springer, Berlin (1992), still offers the best prognostic information in the case of epithelial tumours, because it describes the extent of tumour involvement at the time of diagnosis. Nevertheless, this system has serious limitations, since some patients with early tumour stages develop metastases after curative surgery, even if the tumour does not reappear locally. Other patients with advanced stages of cancer remain disease-free after the surgery. New diagnostic markers for these tumours are clearly needed.

Particularly in colorectal cancer (cancer of the colon or rectum), there are very few diagnostic markers. In principle, further markers might become available if the genotype or phenotype of the tumour cells could be compared with normal cells. However, the interpretation of comparative studies on colorectal cancer has so far proved disappointing, because of inter-sample variations and methodological problems. For example, comparison of protein fingerprints obtained from tumour cell lines with those obtained from human colonic crypts proved to have no clinical implications, see Ji *et al*., Electrophoresis *15*, 391-405 (1994). Another problem is the physiologically high content of proteases in the intestinal mucosa.

Previously, it has been known from WO-A-9 709 600 a method of isolating cells from a faecal stool via immune-separation with magnetic beads, wherein the stool is cooled to a temperature below its gel freezing point.

### Summary of the invention

It has now been found that a proper comparison can be made between the genotype or phenotype of samples from normal and tumorous or other abnormal tissue, using a procedure which enables epithelial cells to be isolated as cellular material substantially free of stroma (connective tissue) and other impurities. This cell preparation is sufficiently pure to enable differences between the normal and abnormal cells to be identified and used to make protein or nucleic acid maps. These maps have enabled further markers for colorectal cancer to be found. Further, the procedure has a general applicability which extends to other kinds of solid epithelial tumours and other abnormalities found in epithelial cells from a variety of types of body tissue.

The invention thus provides a method of diagnosis of an abnormality in epithelial cells, especially tumours, in a sample of tissue taken from the location of the suspected abnormality, especially tumorous, epithelial cell-containing tissue of a patient, especially a human patient. The method comprises (consists of or includes) size-separating stroma from epithelial cells, separating the epithelial cells from most of the other tissues still present, by reacting the tissue with a monoclonal antibody specific for epithelial cells, collecting the reacted cells bound to the antibody and releasing them from the antibody, whereby the sample contains at least 90 percent epithelial cells by volume of the total tissue of the sample and comparing at least one phenotypic or genotypic characteristic of the sample with corresponding normal epithelial cells. Preferably the basis for determining that the purified sample contains at least 90 percent epithelial cells by volume of total tissue of the sample is by fluorescence-activated cell sorting using anti-cytokeratin antibodies after membrane permeabilization of the cells.

By use of the above method, two dimensional gel electrophoretic maps have been produced from the epithelial cells of normal and colorectal tumour-bearing patients and compared. This has resulted in the finding that certain protein spots are over-produced or under-produced in colorectal tumour cells.

### Brief description of the drawings

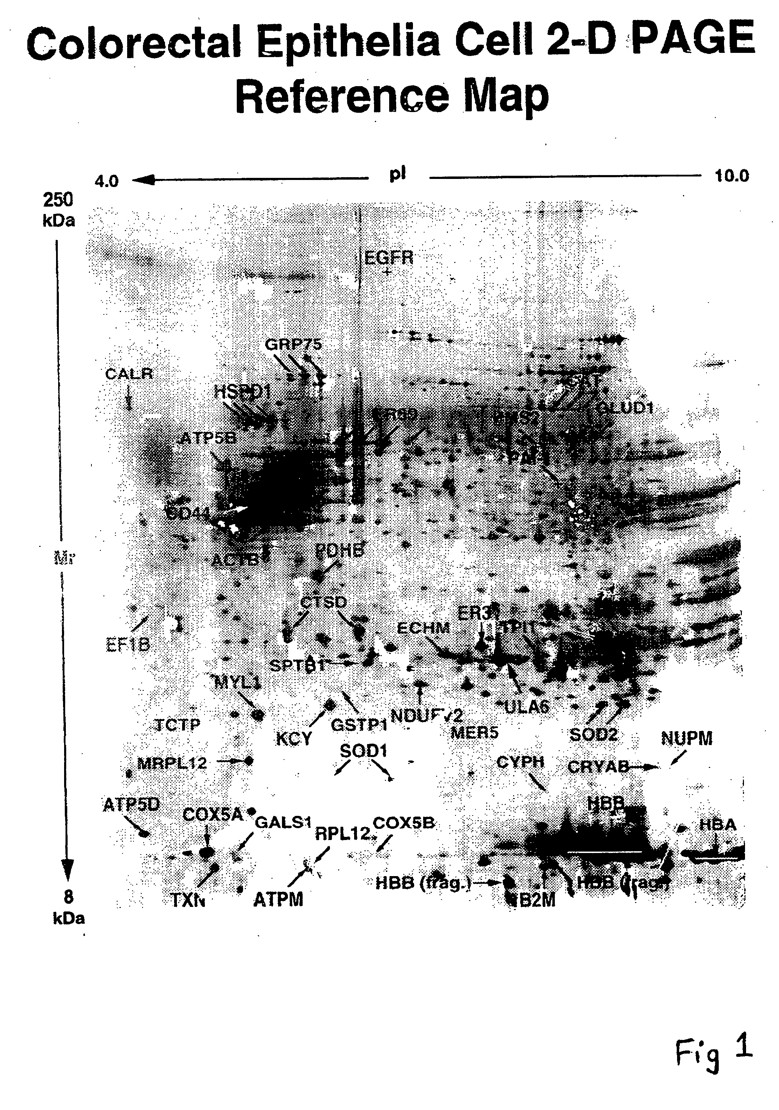

Fig. 1 is a 2D-gel map of proteins present in normal colorectal epithelial cells of a cell preparation produced by the method of the invention.

### Description of the preferred embodiments

While the invention relates principally to colorectal tumours, it is applicable to any other solid tumour containing epithelial cells, in particular to lung, breast, stomach, pancreas, prostate and kidney tumours, for example. Further, it is applicable to detecting other abnormalities of epithelial cells, such as are associated with rectal colitis, Crohn's disease or diverticulitis, for instance.

The most preferred procedure of the invention for separating the epithelial cells sufficiently to form the highly pure cell preparation comprises reacting the tissue with a monoclonal antibody specific for epithelial cells and collecting the reacted cells. The monoclonal antibody can be any which reacts with an appropriate epithelial cell surface protein, especially a receptor. A particularly preferred such antibody is Ber-EP4, as described by U. Latza, J. Clin. Pathol. 43, 213 (1990). This antibody is available in the form of magnetic beads ("Dynabeads") coated with it. Since the magnetic beads can be readily separated magnetically, in the usual way in assay technology, the epithelial cells can be collected magnetically and then washed from the beads.

Another way of separating the cells would be by a non-magnetic heterogeneous method using the same or another ligand such as a monospecific antibody or a peptide which binds strongly to an epithelial cell receptor. The ligand is bound to a solid phase, which is subsequently washed to remove the epithelial cells which have become bound thereto.

Conveniently, the preparation of epithelial cells is used to determine a phenotypic difference between normal and abnormal cells. This may be in terms of the presence of a greater or smaller concentration in the abnormal cells than in the normal cells. This possibility includes the complete absence of a protein from one or the other.

The invention is useful in its own right for tissue mapping, to show differences between normal and abnormal tissue, whether these differences have a genetic origin or not and whether or not they have any value in relation to prognosis of the disease. Two particularly preferred methods of mapping are protein and nucleic acid mapping. Proteins can conveniently be mapped by two dimensional gel electrophoresis. The spots are defined by reference to a 2D-PAGE carried out on a protein extract of the sample by a method comprising
(1) centrifuging 1000 microlitres of the protein extract at 10,000 x G at room temperature for 5 minutes to form a pellet, dissolving the pellet in 300 microlitres of a solution containing 8M urea, 40% CHAPS, 40 mM Tris, 65 mM dithioerythritol and a trace of bromophenol blue and loading the resulting sample solution onto 3 mm wide x 180 mm long strips of immobilised pH gradient (IPG) polyacrylamide gel having a non-linear, signoidal (S-shaped) pH gradient;
(2) rehydrating the gel overnight with 25ml of an aqueous solution of 8M urea, 2% w/v CHAPS, 10 mM dithioerythritol and 2% v/v buffers of pH 3.5 to 10 and a trace of bromophenol blue;
(3) loading the sample solution onto the IPG gel strips for first dimension electrophoresis and running the electrophoresis at a voltage which is increased linearly from 300 to 3500 V during 3 hours, then for 3 additional hours at 3500 V, and finally for 17 hours at 5000 V;
(4) treating the IPG gel strips with 100ml of an aqueous solution containing 50 mM Tris-HCl, pH 6.8, 6M urea, 30% w/v glycerol, 2% w/v SDS, 2.5% v/v iodoacetamide and a trace of bromophenol blue for 5 minutes, to resolubilize the proteins and reduce any -S-S- bonds;
(5) preparing a polyacrylamide gradient slab gel 160 x 200 x 1.5mm from an aqueous solution containing 9 to 16% acrylamide monomer, 2.6% diacryloylpiperazine crosslinking agent, 5 mM sodium thiosulfate, 0.05% tetramethylethylenediamine, 0.1% ammonium persulfate and 0.375M Tris-HCl buffer, pH 8.8, all percentages being w/v, overlayering with sec-butanol for two hours, replacing the sec-butanol with water and standing for 15 hours, overlayering the slab gel with an aqueous solution containing 0.5% agarose, Tris-glycine-SDS at pH 8.3, at 25 mM Tris, 198 mM glycine and 0.1% SDS, heated to 70°C and loading the IPG gel strips thereon for second dimension electrophoresis;
(6) running the second dimension electrophoresis at a constant 40 mA/gel and at 10°C for 5 hours; and
(7) silver staining the gels and scanning them with a laser densitometer, to give a computer-generated image of the stained gel.

RNA can be mapped by amplifying the cellular RNA (preferably total cellular or total cytoplasmic; mRNA is much more difficult to extract from some samples) and running the resultant amplified nucleic acid (conveniently as DNA) on a gel to provide a characteristic band pattern (so-called fingerprint). Most preferably the two maps are correlated, in a search for a protein which is over-produced or under-produced in tumour cells and is accompanied by an increase or decrease in RNA production.

Preferably, the invention is used to detect and test potential new markers for cancers of various kinds, according to the tissue of origin. Once a marker has been thus detected, it may well be possible to eliminate some of the steps of sample preparation and simply compare the tumorous and normal tissue for the marker. However, whether this is possible will depend on the individual markers.

The markers may be detectable as proteins by any of the conventional means, including two dimensional gel-electrophoresis, western blotting with an antibody against the protein, a combination of western blotting with one dimensional gel-electrophoresis, immunoassay (especially enzyme-linked, enhanced chemiluminescent assay).

A method of assay for presence or amount of the marker in a sample from a patient preferably comprises (consists of or includes) a gel electrophoretic method or immunological method or some combination of the two. Conveniently, the method of testing is wholly or predominantly immunological, i.e. typically it involves interaction between the protein of the invention and an antibody thereto or between an antibody of the invention and another antibody. Immunological methods preferably comprise Enzyme-Linked Immuno-Sorbent Assay (ELISA) or western blotting (also referred to as immunoblotting).

For immunological assay, antibody is required. To obtain antibody, it is first necessary to purify and identify at least one protein of the marker spots or a protein sufficiently immunologically similar thereto as to have the same specific epitope. The protein may be purified by standard methods. Thus, the spot is excised from the 2D-gel (or electroeluted into solution or electro-transferred to a membrane and excised from the membrane) and, de-stained by a known method.

The purified protein can then be sequenced by any of the well-known methods, including N-terminal sequencing by Edman degradation (or, if the N-terminus of the protein is blocked, applied to a fragment cleaved by CNBr or by digestion with a peptidase). Mass spectrometry may also be used for sequence determination, especially the method of M. wilm et al., Nature 379, 466-469 (1996). After the protein has been sequenced, it can be checked in sequence databases for similarity to other known proteins. If the protein is already known or very similar to one which is already known, an antibody will probably be commercially available. Otherwise, an antibody will have to be raised for the purposes of an immunological method of assay of the marker protein.

The purified protein can be used directly to raise antibodies or a synthetic protein or peptide thereof could be made by recombinant DNA means, using a pool of labelled degenerate oligos as a probe or primer.

With knowledge of the full amino acid sequence of the protein, various peptides thereof or even the full length of protein can be synthesised by the usual methods of peptide synthesis. Peptides thereof can be tested for reaction with the said polyclonal antibodies raised against the full length protein. These peptides which give a reaction can then be synthesised and used in place of the full length protein to raise antibodies or in competition or displacement assays for the protein present in the sample.

The term "antibody", as used herein, includes polyclonal, monoclonal antibodies, fragments of antibodies such as Fab and genetically engineered antibodies. The antibodies may be chimeric or of a single species.

Although the antibodies raised initially may be polyclonal, monoclonal antibodies can be prepared using the well known Köhler-Milstein method. Conveniently, the ascites fluid from mouse-mouse hybridomas is used and screened against the proteins purified from spots cut from a preparative gel.

Antibodies may also be raised by expressing the immunoglobulin gene on the surface of a bacteriophage and screening the resultant clones against the specific antigen, i.e. the marker protein of the present invention. See, e.g., S.L. Morrison in "Molecular Biology and Biotechnology" ed. Robert A Meyers, VCH Publishers Inc. 1995, at page 37, the disclosure of which is herein incorporated by reference.

Protocols for western blotting are well known. After transfer of the protein from the electrophoretic gel, which may be a 1D-gel or a 2D-gel, to a suitable membrane, preferably of nitrocellulose or polvinylidene difluoride, the membrane is blocked to prevent nonspecific adsorption of immunological reagents. Typical blocking solutions are of skimmed milk powder or bovine serum albumin. After blocking, the protein can be detected directly or indirectly. Direct detection uses labelled primary antibodies, while in indirect detection a second antibody is raised against the first and the second antibody is labelled. The antibodies are usually labelled with an enzyme such as peroxidase or alkaline phosphatase or with a ligand which has a high affinity for a co-ligand, such as biotin, which has a high affinity for streptavidin and avidin. The protein is then detected by adding an enzyme substrate, conveniently a color-forming one, to read out the enzyme label or an enzyme-labelled co-ligand plus a substrate for the enzyme if a high affinity ligand type of label was used.

An alternative method of quantifying or detecting the presence of the protein is the use of immunoassays, preferably ELISAs, which may be performed on the epithelial cell sample or on protein, isolated or partly isolated by ID or 2D-gel electrophoresis, transferred to a membrane by blotting. Types of immunoassay useful in this invention include antibody capture assays, antigen capture assays (also called competition or displacement assays) and the two antibody sandwich immunoassay. All the immunoassays require labelled marker protein, antibodies or secondary reagents for detection or quantitation. The labels described above for western blotting may be used. The labels may be "read out" or detected by any conventional method, e.g. in an ELISA by using color-forming, fluorescent or chemiluminescent means. Enhanced chemiluminescent assays are particularly preferred and several commercial kits for such assays are known in connection with peroxidase and alkaline phosphatase enzyme labels.

Test kits appropriate to the above and other forms of assay will be apparent to those skilled in the art. Preferred such kits are those for an antibody capture assay and comprise a first an antibody to a protein marker and a labelled second antibody thereto, provided either separately or in the form of a linked double antibody. The test kits may optionally include any of a variety of supports or solid phases, especially plastic tubes and microtiter plates. Procedures for assisting the binding of antigens or antibodies to such supports are well known.

Although heterogeneous assays are cheap and well understood in the art, homogeneous assays may also be used in this invention.

Immuno-PCR may be used as a method of amplifying the signal of an immunoassay described above. In this technique the antibody is covalently linked to a piece of arbitrary DNA comprising PCR primers, whereby the DNA with antibody attached is amplified by the PCR. See Hendrickson *et al*., Nucl. Acids Res. *23*, 522-529 (1995), the disclosure of which is herein incorporated by reference.

It is, of course possible, to assay the marker protein by a method which comprises at least one dimension of gel electrophoresis. The gel electrophoresis protocols are not to be regarded as limited to those described herein. They may be varied considerably. The sample is first disrupted, e.g. with a high molar concentration of urea, detergents and dithiothreitol or dithioerythritol to break -S-S- bonds. The first dimension gel is then run in an ampholyte mixture which establishes a pH gradient across the gel, so that the proteins migrate to their isoelectric point, i.e. to a point at which the pH of the gel within the pH gradient established by the ampholytes is equal to the charge on the protein. At this stage, there may be sufficient resolution of the marker spots from others to provide an adequate test for the tissue abnormality. If not, a western blot or immunoassay, using antibody to the protein of the invention, may be performed. Alternatively, the second dimension electrophoresis may then be performed. Here the first dimension gel is loaded, transversely to the direction of current, onto a second gel. This is normally an SDS-PAGE gel, the principle being that charges on the protein are swamped by the effect of the SDS, so that the gel separates the proteins according to molecular weight. This is aided by a higher acrylamide concentration.

Abnormal genotypes may be detectable as RNA by any of the conventional means for detecting cellular RNA, especially probing with labelled DNA specific for the RNA, preferably at two sites in the DNA encoding the protein so as to increase the stringency of binding of the probe to the sample. Labelled oligonucleotides of length, say 17 to 40 nucleotides are usual for this purpose. Preferably the RNA extracted from the sample would be amplified, e.g. by polymerase chain reaction, before carrying out this assay. Since the sample RNA will usually have to be amplified anyway, it may be more convenient to use a labelled amplification procedure, such as PCR using a labelled primer. In this connection, it has been found that a random arbitrary primer can be very useful where the genotype of the disease state is unknown.

The following Examples illustrate the invention.

### Example 1

### Materials and Methods

### Isolation of human colonic crypts

Immediately after human bowel resection surgery for various medical reasons, the surgical specimen was stored on ice. After washing with iced phosphate buffered saline (PBS) solution, a fragment of mucosa (5 x 5cm) was dissected from a healthy portion of tissue, as far removed as possible from the site of disease. The mucosa was then immersed in an ice-cold PBS solution containing EDTA 3mM and a freshly prepared cocktail of proteases inhibitors (leupeptine 50 micrograms/ml, PMSF 0.2 mM and benzamidine 0.8 mM). Crypts were scraped away from the basal membrane with a scalpel, and then gently pressed through a steel mesh having a pore size of 300 micrometres to separate epithelial cells from stroma. The cell suspension obtained in this way was then filtered through a nylon mesh having a pore size of 200 micrometres. This permitted the isolation of single, or small clusters or epithelial and other cells (lymphocytes, macrophages, etc). The stroma (connective tissue) was not altered by this mechanical procedure and could theoretically be used for further analysis. Isolation of epithelial cells

After washing and centrifuging the cells at 350 x g for 10 min at 4°C, they were resuspended in the above mentioned solution at a concentration of 2 x 10⁷ cells/ml. This suspension was then incubated at 4°C for 30 minutes with anti-Epithelial Cell "Dynabeads" coated with Ber-EP4 antibodies in accordance with the instructions of the manufacturer (Deutsche Dynal GmbH, Hamburg, Germany). The cells were then collected with a magnetic particle concentrator (MPC-1, Deutsche Dynal GmbH, Hamburg, Germany) and washed five times. A cell count and viability check were performed using trypan blue and showed over 90% viable cells. This first part of the procedure required no more than 90 minutes.

### Control

The nature of the cell samples was assessed after labelling the preparation products with fluorescein-conjugated anti-cytokeratin antibodies (CAM 5.2, Perkin-Elmer, Norwalk, Connecticut, USA) for 45 min, in accordance with the instructions of the manufacturer. The samples were then sorted qualitatively with a Fluorescence-Activated Cell Sorter (Epics, Coulter-Immunotech, Hamburg, Germany). It was thus estimated that the sample contained at least 90% epithelial cells by volume of total tissue of the sample at that stage.

### Denaturation

After centrifugation, the pellets were denaturated with urea 8M, CHAPS (4% w/v), Tris (40mM) and DTE (65 mM) in accordance with SWISS-2DPAGE protocols, Sanchez et al., Electrophoretics 16, 1131-1151 (1995), and stored at -20°C.

### Analytical 2-D PAGE

Total protein in each sample was assayed as described by the well known Bradford method. 100 micrograms of normal human colonic epithelial cell protein samples were separated by 2-D PAGE. A commercial sigmoidal immobilized pH gradient (IPG) ranging from pH 3.5 to 10.0 (18cm) was used for the first dimension. After equilibration, the IPG gel strips were transferred onto vertical gradient slab gels (9-16% acrylamide) for the second dimension, and run with the Laemmli-SDS-discontinuous system. The crosslinker used was piperazine diacrylyl (PDA 2.6%), and the adjunct catalyst sodium thiosulfate (5mM). A full description of the method is given in the Summary of the Invention above.

### Preparative 2-D PAGE

Three mg of normal protein samples were separated by 2-D PAGE and electro-transferred onto PVDF membranes. A commercial immobilized pH gradient (IPG) ranging from pH 3.5 to 10.0 was used for first dimensional separation. In-gel sample rehydration was used, which enabled a higher sample loading, increased resolution and shorter running time. After equilibration, the IPG gel strips were transferred onto vertical gradient slab gels (9-16% acrylamide) for the second dimension. Electroblotting onto PVDF membranes was then effected using a home-made semi-dry apparatus with 10% methanol and 10 mM CAPS as buffer (pH 11) at 200 V for 2 h. The membranes were stained with amido black.

### Identification of proteins

### Gel matching

After the laser densitometer scanning, 2-D PAGE image analysis was carried out using the MELANIE II software package (Bio-Rad, CA). Protein spots were detected and quantified automatically. The optical density, area and volume were computed and directly related to protein concentration. The relative optical density and relative volume were also calculated to correct for differences in gel loading and staining. The experimental pI and MW of the spots, as well as protein identification, were deduced by the MELANIE II image analysis software (Bio-Rad, Hercules, California, USA) from the liver SWISS-2DPAGE reference map, Sanchez *et al*. (1995) referred to above.

### Microsequencing

PVDF membranes were stained with Amido Black, destained with water, and dried. The spots of interest were excised, dried under nitrogen, and kept in Eppendorf tubes at -20°C until microsequencing was performed. Ten to fifteen Edman degradation cycles were performed for each spot and the SWISS-PROT database, see Bairoch *et al*., Nucleic Acids Res. *22*, 3578-3580 (1994) was searched to establish the identity of already known proteins.

### Immunoblotting

After electrophoretic transfer onto PVDF membranes, the latter were blocked with 10 mM Tris-HCl (pH 7.0), 500 mM NaCl, 0.05% Tween-20 and 0.5% non-fat dry milk prior to incubation with the primary antibodies. Horseradish peroxidase-conjugated goat anti-rabbit and antimouse antibodies were used at a 1/1000 dilution as secondary antibodies. Blots were developed using enhanced chemiluminescence and X-ray film as described by Boehringer Mannheim. A monoclonal antibody anti-TCTP was used as an internal marker for control and comparison with reference maps.

### (5) Results

The above-described size-separation and magnetic separation using "Ber-EP4" coated "Dynabeads" permitted enrichment of the samples to over 95% epithelial cells. This pure state was assessed by a second consecutive staining using fluorescein-conjugated anti-cytokeratin antibodies followed by Fluorescence Activated Cell Sorting. A quality control using scanning electron microscopy showed no degradation of the cell surfaces. Contamination by blood, as assessed by serum albumin, was low after washing and enrichment. Comparison of samples before and after separation with the "Dynabeads" showed an improvement in contrast and disappearance of degradation patterns. A mean number of 900-1200 spots/gel was obtained using silver staining.

Spots relating to 40 proteins were sequenced and identified as listed in Table 1. A protein master map of the normal colonic epithelial cells was defined (Figure 1). A key to Figure 1 is provided in the following Table, in which all pI and molecular weight values are apparent (not absolute). Using immunoblotting, expression of proteins having a possible diagnostic or prognostic significance in tissue abnormalities such as colorectal cancer was assessed. In particular, protein families CD44, Mn-SOD, EGF-receptor, PAI-1, hsp70 and PMS2 were localized.

Examples of references relating to the implication of the above proteins in the diagnosis or prognosis of tissue cell abnormalities are as follows:
EGFR: R. Seshadri *et al*., Int. J. Cancer, *69*, 23-27 (1996)
CD44: L. H. Finke *et al*., The Lancet, *345*, 583 (1995); J. Mulder, The Lancet *344*, 1470-1472 (1994); Z. Nihei *et al*., Surgery Today, 26, 760-761 (1996)
PMS2: B. Liu *et al*., Nature Medicine, *2*, 169-174 (1996)
MnSOD: A.M.L. Janssen *et al*., Abstract No. 1327, Proceedings of the 18th Meeting of the American Association for Cancer Research, Washington D.C., April 20-24, 1996, *37*, 194-195 (1996);
I. Katsumi *et al*., JICST Abstract Accession No. 97A0705439 (1997);
HSP-70: J. Stulik *et al*., Electrophoresis *18* (3-4), 625-628 (1997).

Similar work can be carried out on chopped pieces of tumour tissue to yield gels which produce spots representing proteins which are over- or under-expressed in the tumour tissue. Comparison of the maps, especially using MELANIE II software, enables proteins of diagnostic or prognostic significance to be identified. Examples are given in a co-pending PCT Patent Application of Electrophoretics International plc et al. filed at the UK Receiving Office on 23rd March 1998, claiming the same priority, and entitled "Diagnosis of Colorectal Cancer and Proteins and Antibodies for Use Therein", the disclosure of which is herein incorporated by reference.

The preparation of the sample to enrich it in epithelial cells has the advantage that reproducible patterns can be obtained even when the stroma (the intercellular connective tissue) differs in different samples. In this way, samples are freed from blood, stool material, lymphocytes etc.

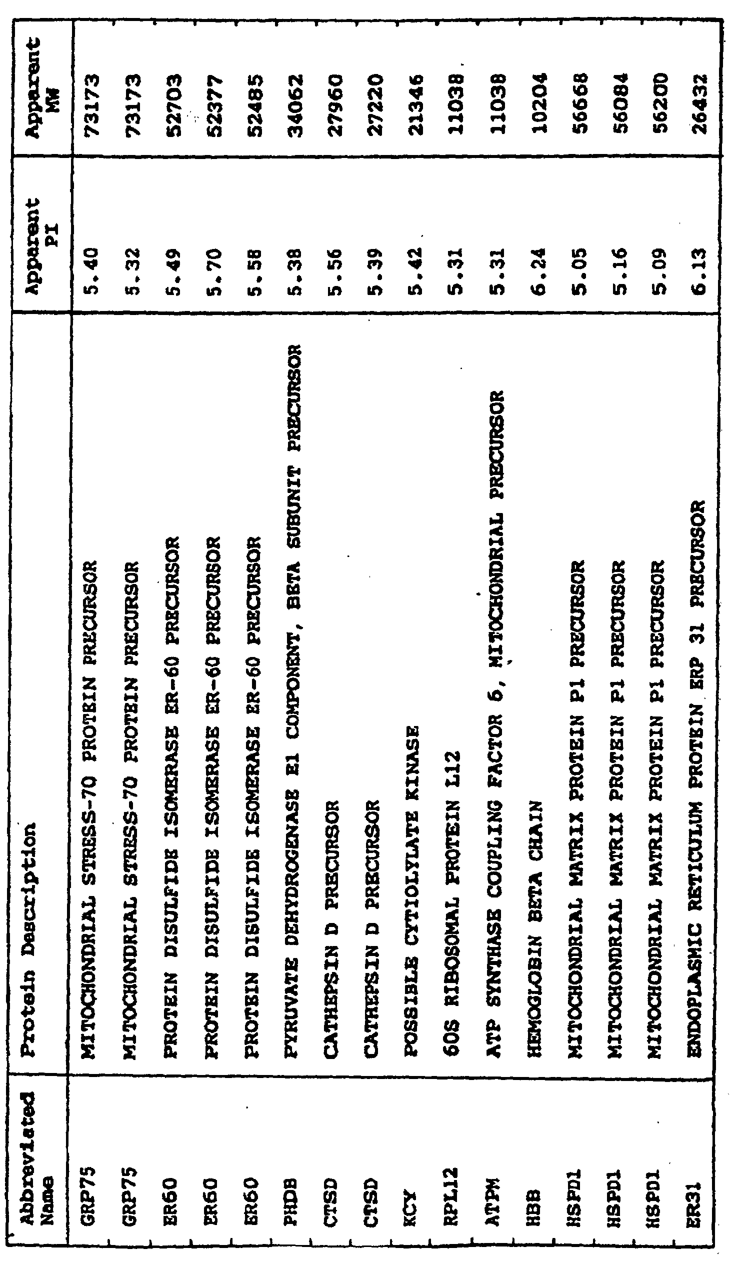

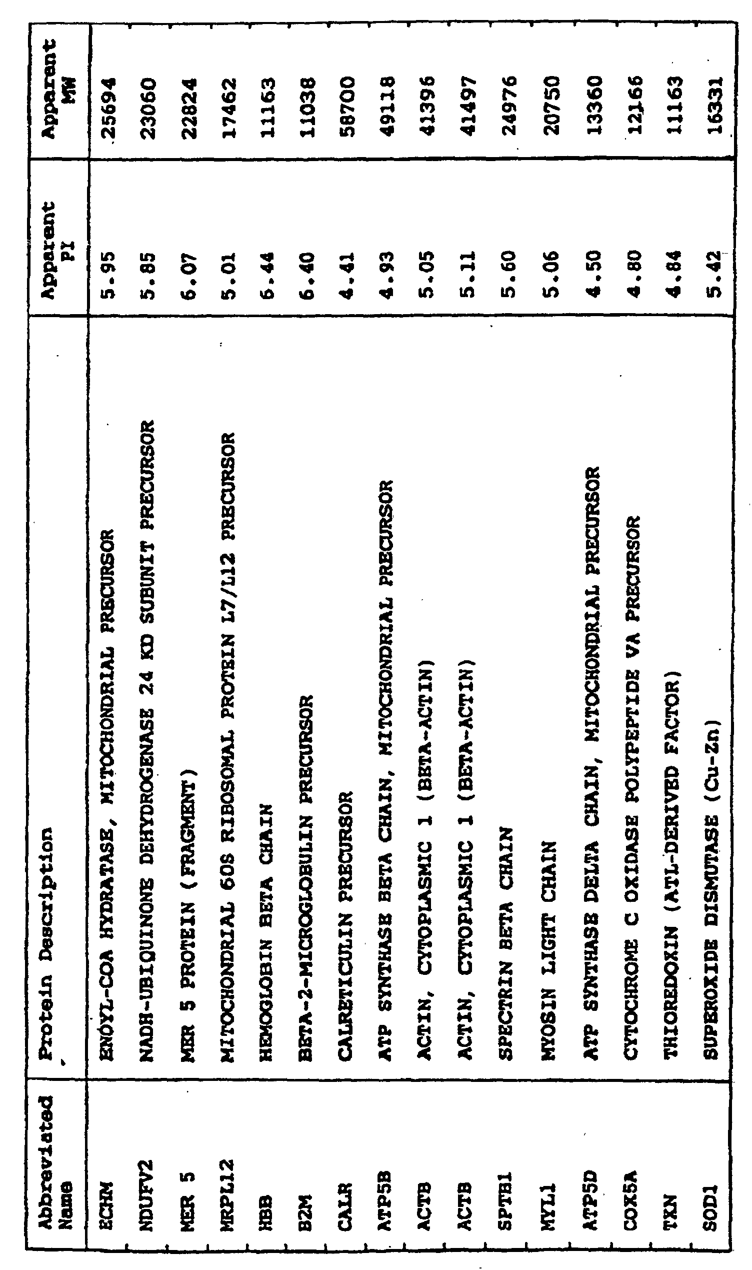

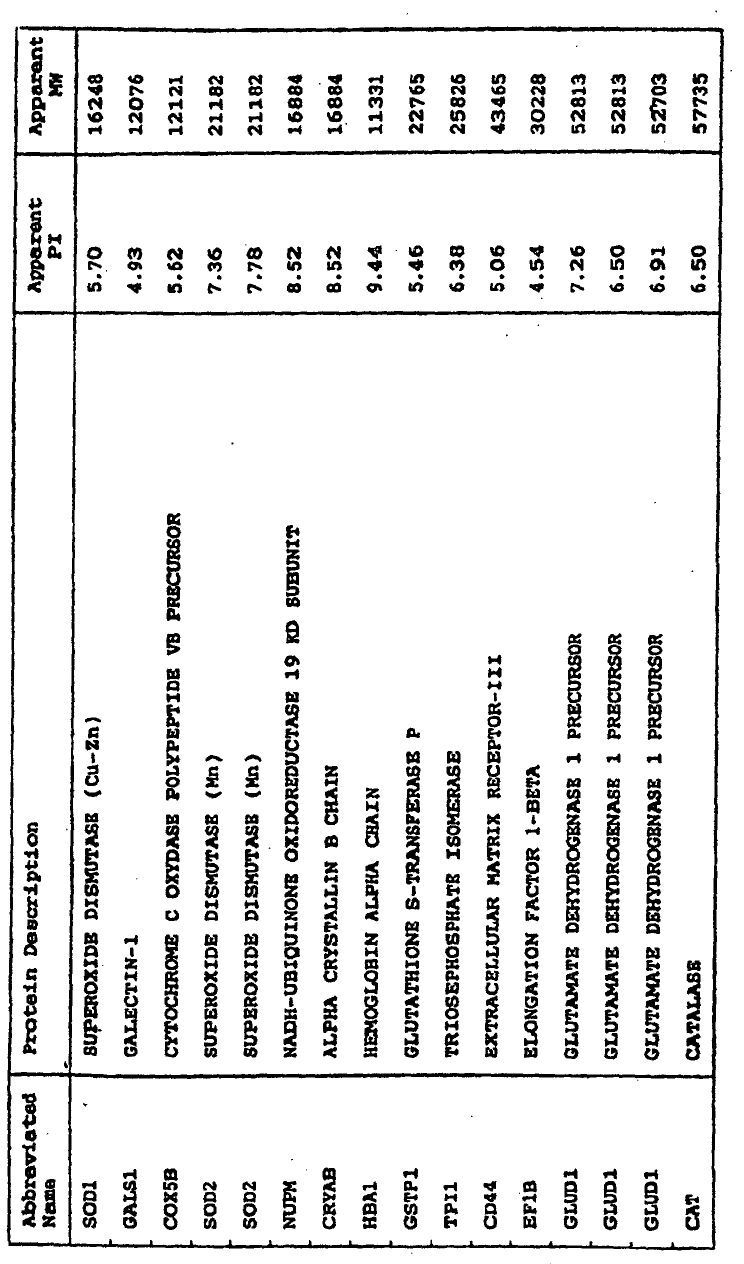

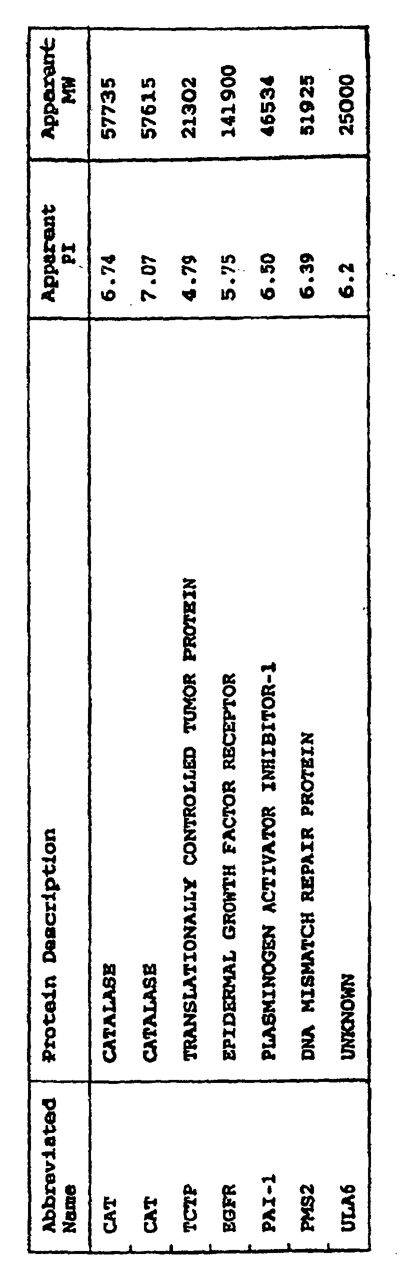

### Example 2

### Samples

Samples were taken from tumorous and normal human colorectal tissue. Epithelial cells from this colorectal tissue were isolated with magnetic particles ("Dyna-Beads"), as described in Example 1.

### RNA Extraction

Total cellular RNA was extracted by the acid guanidinium-thiocyanate-phenol-chloroform extraction method of Chomczynsky and Sacchi, Analytical Biochemistry *162*, 156-159 (1987). In summary, the procedure was as follows. To the tissue, minced and homogenised on ice, were added a 4M guanidinium thiocyanate solution containing 2-mercaptoethanol, followed by sodium acetate, pH 4, phenol and chloroform. After thorough mixing and centrifugation, the aqueous phase containing the RNA was removed and the RNA precipitated with isopropanol or ethanol. After centrifuging, the RNA pellet was washed with ethanol and dissolved in SDS.

Total cytoplasmic RNA was extracted as described by Aubel et al, "Current Protocols in Molecular Biology" (1981), Unit 4.1, "Preparation of Cytoplasmic RNA from Tissue Culture Cells". In summary, the procedure was as follows. Cells were washed with ice-cold phosphate-buffered saline and kept on ice for all subsequent manipulations. The pellets or harvested cells were resuspended in a lysis buffer containing the nonionic detergent "Nonidet" P-40. Lysis of the plasma membranes occurs almost immediately. The intact nuclei were removed by a brief microcentrifuge spin, and sodium dodecyl sulfate was added to the cytoplasmic supernate to denature protein. Protein and lipid were removed by extractions with phenol/chloroform and chloroform. In some cases, it was necessary to treat the cytoplasmic extract with protease prior to extraction. The cytoplasmic RNA was recovered by ethanol precipitation and quantitated by measuring its absorbance at 260 and 280 nm.

### Primer Selection

Arbitrary oligonucleotide (DNA) primers were used. They have not yet been optimised, but will normally have from 17 to 40 nucleotides.

### Assay conditions

The general method was as follows. All RNAs were reverse transcribed to cDNA using an arbitrary primer, after which PCR was performed with the same primer from the product of the previous reaction in 5 cycles at a low stringency annealing temperature and 35 cycles at a high stringency temperature, all this in the presence of a radioactive nucleotide so that the products could be resolved in a sequencing type gel.

In more detail, each reverse transcription reaction (RT) was performed in a final volume of 20 µl using 50 ng of total RNA, 50 mM Tris-HCl (pH 8.3), 75 mM KCl, 3 mM MgCl₂, 10 mM DTT, 0.5 mM of each dNTP, 0.5 µM primer, 20u RNAse inhibitor and 200u reverse transcriptase (Gibco BRL). Incubation with the enzyme was performed for 1 h at 37°C and reaction was stopped by heating it at 95°C for 5 min.

Subsequent PCRs were performed with 2 µl of the product of the RT, 10 mM Tris-HCl, 2.5 mM MgCl₂, 50 mM KCl, 0.1mg/ml gelatin, 0.1 mM of each dNTP, 2µM primer, 1.25u Taq polymerase (Boehringer Mannheim) and 2 µCi [α-³³P]-dATP in a final volume of 25 µl. The reactions consisted of an initial denaturation step (94°C for 1 min), 5 cycles at low stringency conditions (94°C for 1 min; 45 seconds at the annealing temperature; 72°C for 1 min 30 sec), 35 cycles at high stringency conditions (94°C for 1 min; 60°C for 45 sec; 72°C for 1 min) and a final extension (72°C for 5 min). All reactions were performed in a Programmable Thermal Controller-100 (MJ Research Inc.).

PCR products were diluted (1/4) with 95% deionized formamide denaturing loading buffer (DLB), heated at 95°C for 3 min and immediately cooled on ice. Two µl of each diluted sample were loaded in a 6% polyacrylamide / 8M urea denaturing sequencing gel (40 cm long, 30 cm wide, 0.4mm thick) and run at 55 W for 3 to 4 hours. The gels were dried under vacuum at 80°C and exposed to a X-ray film at room temperature, without an intensifier screen, for 1-3 days. The "fingerprints" represented by the gels from the normal and abnormal tissue samples were then compared.

DNA was excised from the gel bands and sequenced. It can then be determined whether it predicts proteins appearing on the 2D-gel.

## Claims

1. A method of diagnosis of an epithelial cell abnormality in a sample of tissue taken from the location of a suspected said abnormality, which comprises size-separating stroma from epithelial cells, separating the epithelial cells from most of the other tissue still present, by reacting the tissue with a monoclonal antibody specific for epithelial cells, collecting the reacted cells bound to the antibody and releasing them from the antibody, whereby the sample contains at least 90 percent epithelial cells by volume of the total tissue of the sample and comparing at least one phenotypic or genotypic characteristic of the sample with corresponding normal epithelial cells.

2. A method according to Claim 1, wherein the size-separation is effected by filtering the tissue sample through a filter of about 300 micrometres and collecting the epithelial cells passing through the filter.

3. A method according to Claim 2, wherein the size-separation further comprises further filtering the epithelial cells through a filter of 150-250 micrometres and collecting the epithelial cells passing through the filter.

4. A method according to Claim 1, 2 or 3, wherein a phenotypic characteristic is compared by determining whether at least one protein is present in a greater or smaller concentration in the sample than in the normal cells.

5. A method according to Claim 4, wherein the protein is selected from among those present in a greater or smaller amount in a two dimensional electrophoretic gel of proteins extracted from the sample than from the normal cells.

6. A method according to Claim 5, wherein the two dimensional electrophoretic gel is obtainable by
(1) centrifuging 1000 microlitres of the protein extract at 10,000 G at room temperature for 5 minutes to form a pellet, dissolving the pellet in 300 microlitres of a solution containing 8M urea, 40% CHAPS, 40 mM Tris, 65 mM dithioerythritol and a trace of bromophenol blue and loading the resulting sample solution onto 3mm wide x 180mm long strips of immobilised pH gradient (IPG) polyacrylamide gel having a non-linear, sigmoidal (S-shaped) pH gradient;
(2) rehydrating the gel overnight with 25ml of an aqueous solution of 8M urea, 2% w/v CHAPS, 10 mM dithioerythritol and 2% v/v buffers of pH 3.5 to 10 and a trace of bromophenol blue;
(3) loading the sample solution onto IPG gel strips for first dimension electrophoresis and running the electrophoresis at a voltage which is increased linearly from 300 to 3500 V during 3 hours, then for 3 additional hours at 3500 V, and finally for 17 hours at 5000 V;
(4) treating the IPG gel strips with 100 ml of an aqueous solution containing 50 mM Tris-HCl, pH 6.8, 6M urea, 30% w/v glycerol, 2% w/v SDS, 2.5% v/v iodoacetamide and a trace of bromophenol blue for 5 minutes, to resolubilize the proteins and reduce any -S-S- bonds;
(5) preparing a polyacrylamide gradient slab gel 160 x 200 x 1.5mm from an aqueous solution containing 9 to 16% acrylamide monomer, 2.6% diacryloylpiperazine crosslinking agent, 5mM sodium thiosulfate, 0.05% tetramethylethylenediamine, 0.1% ammonium persulfate and 0.375M Tris-HCl buffer, pH 8.8, all percentages being w/v, overlayering with sec-butanol for two hours, replacing the sec-butanol with water and standing for 15 hours, overlaying the slab gel with an aqueous solution containing 0.5% agarose, Tris-glycine-SDS at pH 8.3, at 25 mM Tris, 198 mM glycine and 0.1% SDS, heated to 70°C and loading the IPG gel strips thereon for second dimension electrophoresis;
(6) running the second dimension electrophoresis at a constant 40 mA/gel and at 10°C for 5 hours; and
(7) silver staining the gels and scanning them with a laser densitometer, to give a computer-generated image of the stained gel.

7. A method according to Claim 1, 2 or 3, wherein a genotypic characteristic is compared by determining whether the total RNA or cytoplasmic RNA is present in a greater or smaller concentration in the sample than in the normal cells.

## Patentansprüche

1. Verfahren zur Diagnose von Epithelzellenanomalien in einer Gewebeprobe, die von der Stelle der mutmaßlichen Anomalie entnommen wurde, umfassend die Größentrennung des Stromas von den Epithelzellen, die Trennung der Epithelzellen von einem Großteil des übrigen, noch vorhandenen Gewebes durch Reaktion des Gewebes mit einem monoklonalen Antikörper, der spezifisch für Epithelzellen ist, Gewinnung der zur Reaktion gebrachten, an den Antikörper gebundenen Zellen und Freisetzung derselben vom Antikörper, wobei die Probe wenigstens 90 Prozent Epithelzellen bezogen auf das Volumen des gesamten Gewebes der Probe enthält, und Vergleichen wenigstens eines phänotypischen oder genotypischen Merkmals der Probe mit entsprechenden normalen Epithelzellen.

2. Verfahren nach Anspruch 1, wobei die Größentrennung durch Filtrieren der Gewebeprobe durch ein Filter mit etwa 300 µm und Auffangen der durch das Filter hindurchgehenden Epithelzellen erfolgt.

3. Verfahren nach Anspruch 2, wobei die Größentrennung zudem das weitere Filtrieren der Epithelzellen durch ein Filter mit etwa 150-250 µm und das Auffangen der durch das Filter hindurchgehenden Epithelzellen umfaßt.

4. Verfahren nach Anspruch 1, 2 oder 3, wobei ein phänotypisches Merkmal verglichen wird durch Bestimmen, ob wenigstens ein Protein in der Probe in höherer oder geringerer Konzentration als in den normalen Zellen vorhanden ist.

5. Verfahren nach Anspruch 4, wobei das Protein ausgewählt ist aus denjenigen, die in einem zweidimensionalen Elektrophoresegel mit Proteinen, die aus der Probe extrahiert wurden, in höherer oder geringerer Menge vorhanden sind als die aus normalen Zellen.

6. Verfahren nach Anspruch 5, wobei das zweidimensionale Elektrophoresegel erhältlich ist durch.
(1) Zentrifugieren von 1000 µl des Proteinextrakts bei 10 000 g und Raumtemperatur für 5 Minuten, so dass sich ein Pellet bildet, Lösen des Pellets in 300 µl einer Lösung, enthaltend 8 M Harnstoff, 40% CHAPS, 40 mM Tris, 65 mM Dithioerythritol und eine Spur Bromphenolblau, und Aufbringen der resultierenden Probenlösung auf 3 mm breite und 180 mm lange Streifen eines Polyacrylamid-Gels mit immobilisiertem pH-Gradienten (IPG), das einen nichtlinearen, sigmoiden (S-förmigen) pH-Gradienten aufweist;
(2) Rehydratisieren des Gels über Nacht mit 25 ml einer wäßrigen Lösung von 8 M Harnstoff, 2% Gew./Vol. CHAPS, 10 mM Dithioerythritol und 2% Vol./Vol. Puffern mit pH 3,5 bis 10 und einer Spur Bromphenolblau;
(3) Aufbringen der Probenlösung auf IPG-Gelstreifen für die Elektrophorese in einer ersten Dimension und Durchführen der Elektrophorese für 3 Stunden bei einer Spannung, die linear von 300 auf 3500 V erhöht wird, dann für weitere 3 Stunden bei 3500 V und schließlich für 17 Stunden bei 5000 V;
(4) Behandeln der IPG-Gelstreifen für 5 Minuten mit 100 ml einer wäßrigen Lösung, enthaltend 50 mM Tris-HCl, pH 6,8, 6 M Harnstoff, 30% Gew./Vol. Glycerin, 2% Gew./Vol. SDS, 2,5% Vol./Vol. Iodacetamid und eine Spur Bromphenolblau, um die Proteine wieder löslich zu machen und etwaige -S-S--Bindungen zu reduzieren;
(5) Herstellen eines tafelförmigen Polyacrylamid-Gradientengels mit 160 × 200 × 1,5 mm aus einer wäßrigen Lösung, enthaltend 9 bis 16% Acrylamid-Monomer, 2,6% Diacryloylpiperazin-Vernetzer, 5 mM Natriumthiosulfat, 0,05% Tetramethylethylendiamin, 0,1% Ammoniumpersulfat und 0.375 M Tris-HCl-Puffer, pH 8,8, wobei alle Prozentanteile in Gew./Vol. sind, Überschichten mit sec-Butanol über zwei Stunden, Ersetzen von sec-Butanol durch Wasser und Stehenlassen für 15 Stunden, Überschichten des tafelförmigen Gels mit einer wäßrigen Lösung, enthaltend 0,5% Agarose, Tris-Glycin-SDS, pH 8,3, mit 25 mM Tris, 198 mM Glycin und 0,1% SDS, erwärmt auf 70°C, und Aufbringen der IPG-Gelstreifen für die Elektrophorese in der zweiten Dimension;
(6) Durchführen der Elektrophorese in der zweiten Dimension bei konstant 40 mA/Gel und 10°C für 5 Stunden; und
(7) Silber-Anfärbung der Gele und Abtastung derselben mit einem Laserdensitometer, um ein computererzeugtes Abbild des angefärbten Gels zu ergeben.

7. Verfahren nach Anspruch 1, 2 oder 3, wobei ein genotypisches Merkmal verglichen wird durch Bestimmen, ob die Gesamt-RNA oder zytoplasmatische RNA in der Probe in höherer oder geringerer Konzentration als in den normalen Zellen vorhanden ist.

## Revendications

1. Procédé de diagnostic d'une anomalie de cellule épithéliale dans un échantillon de tissu prélevé à l'emplacement de la dite anomalie présumée, qui comprend la séparation par tailles du stroma des cellules épithéliales, la séparation des cellules épithéliales de la plupart des autres tissus encore présents, en faisant réagir le tissu avec un anticorps monoclonal spécifique aux cellules épithéliales, recueillant les cellules ayant réagi qui sont liées à l'anticorps et en les libérant de l'anticorps, dans lequel l'échantillon contient au moins 90 pour cent en volume de cellules épithéliales rapporté au tissu total de l'échantillon, et en comparant au moins une caractéristique phénotypique ou génotypique de l'échantillon avec des cellules épithéliales normales correspondantes.

2. Procédé selon la revendication 1, dans lequel la séparation par tailles est réalisée en filtrant l'échantillon de tissu à travers un filtre à environ 300 µm et en recueillant les cellules épithéliales passant à travers le filtre.

3. Procédé selon la revendication 2, dans lequel la séparation par tailles comprend en plus une filtration complémentaire des cellules épithéliales à travers un filtre à 150-250 µm et un recueil des cellules épithéliales passant à travers ce filtre.

4. Procédé selon la revendication 1, 2, ou 3, dans lequel on compare une caractéristique phénotypique en déterminant si au moins une protéine est présente à une concentration dans l'échantillon supérieure ou inférieure à celle des cellules normales.

5. Procédé selon la revendication 4, dans lequel on sélectionne la protéine parmi celles qui sont présentes en une quantité supérieure ou inférieure dans un gel d'électrophorèse bidimensionnelle de protéines extraites de l'échantillon à celle des cellules normales.

6. Procédé selon la revendication 5, dans lequel le gel d'électrophorèse bidimensionnelle peut être obtenu en :
(1) centrifugeant 1000 microlitres de l'extrait protéinique à 10 000 G, à température ambiante, pendant 5 minutes pour former une boulette, en dissolvant la boulette dans 300 microlitres d'une solution contenant 8 M d'urée, 40 % de CHAPS, 40 mM de Tris, 65 mM de dithioérythritol et un soupçon de bleu de bromophénol et en appliquant la solution d'échantillon obtenue sur des bandes de 3 mm de large sur 180 mm de long de gel de polyacrylamide à gradient de pH immobilisé (IPG) ayant un gradient de pH non linéaire, sigmoïde (en forme de S),
(2) réhydratant le gel pendant une nuit avec 25 ml d'une solution aqueuse d'urée à 8 mM, 2 % p/v de CHAPS, 10 mM de dithioérythritol et 2 % v/v des tampons de pH 3,5 à 10 et un soupçon de bleu de bromophénol,
(3) appliquant la solution d'échantillon sur les bandes de gel IPG pour l'électrophorèse de première dimension et en réalisant l'électrophorèse en augmentant la tension de manière linéaire de 300 à 3500 V pendant 3 heures, puis en la maintenant à 3500 V pendant 3 autres heures et enfin pendant 17 heures à 5000 V,
(4) traitant les bandes de gel IPG pendant 5 minutes avec 100 ml d'une solution aqueuse contenant 50 mM de Tris-HCl de pH 6,8, 6 M d'urée, 30 % p/v de glycérol, 2 % p/v de SDS, 2,5 % v/v de iodoacétamide et un soupçon de bleu de bromophénol, afin de resolubiliser les protéines et de réduire toutes liaisons -S-S-,
(5) préparant une plaque de gel de polyacrylamide à gradient de 160 x 200 x 1,5 mm à partir d'une solution aqueuse contenant 9 à 16 % de monomère acrylamide, 2,6 % d'agent de réticulation diacryloylpipérazine, 5 mM de thiosulfate de sodium, 0,05 % de tétraméthyléthyiènediamine, 0,1 % de persulfate d'ammonium et 0,375 M de tampon Tris-HCl de pH 8,8, tous les pourcentages étant rapportés au poids/volume, en recouvrant les bandes avec du sec-butanol pendant 2 heures, en remplaçant le sec-butanol par de l'eau pour la maintenir pendant 15 heures, en recouvrant la plaque de gel avec une solution aqueuse contenant 0,5 % d'agarose, du Tris-glycine-SDS à pH 8,3 contenant 25 mM de Tris, 198 mM de glycine et 0,1 % de SDS, chauffée à 70° C, et en déposant les bandes de gel IPG là-dessus pour l'électrophorèse de seconde dimension,
(6) en effectuant l'électrophorèse de seconde dimension à un ampérage constant de 40 mA/gel et à 10° C pendant 5 heures, et
(7) en colorant à l'argent les gels et en les analysant à l'aide d'un densitomètre à laser, pour donner une image produite par ordinateur du gel coloré.

7. Procédé selon la revendication 1, 2, ou 3, dans lequel on compare une caractéristique génotypique en déterminant si l'ARN total ou l'ARN cytoplasmique est présent dans l'échantillon en une concentration supérieure ou inférieure à celle des cellules normales.
